# NEW EUROPEAN PATENT SPECIFICATION

(11) **EP 0 120 301 B2**
(45) Date of publication and mention of the opposition decision: **14.04.1999**
(45) Mention of the grant of the patent: 16.05.1990
(21) Application number: 84101909.4
(22) Date of filing: 23.02.1984
(51) Int. Cl.: A01N 59/12

(54) **Method of producing standardized iodophor preparations and such preparations**
Verfahren zur Herstellung von standardisierten Iodophorpräparaten und solche Präparate
Procédé pour produits des préparations d'iodophores standardisées et de telles préparations

(30) Priority: 02.03.1983 DE 3307219; 15.04.1983 DE 3313655
(43) Date of publication of application: 03.10.1984
(73) Proprietor: Euro-Celtique S.A., Luxemburg (LU)
(72) Inventor: Rackur, Helmut, Dr., D-6250 Limburg (DE); Pinter, Erwig, Dr., D-6250 Limburg (DE); Halpern, Alfred, Dr., Great Neck, NY 11020 (US); Miller, Ronald B., Dr., CH-4059 Basel (CH); Sackler, Mortimer D., Dr., Schattdorf, Uri (CH); Sackler, Raymond R., Dr., Greenwich, CT 06830 (US); Sackler, Richard S., Dr., Greenwich, CT 06830 (US)
(74) Representative: Maiwald, Walter, Dr. Dipl.-Chem.

(56) References cited:
- DE-A- 2 438 594
- DE-A- 2 540 170
- DE-A- 2 718 385
- GB-A- 2 060 385
- GB-A- 2 084 875
- US-A- 3 028 300
- US-A- 4 113 857
- Gmelin, "Jod", pp. 545-549, 556
- Affidavit of Winicov, M.W.
- O. WYSS, F.B. STRANDSKOV, Archives of Biochemistry, 6, 1945, pp. 261-268
- W. SCHMIDT, M. WINICOV, Soap and Chemical Specialties, 1967, pp. 61-64
- W. GOTTARDI, 7th Symposium of the "Österreichische Gesellschaft für Hygiene, Mikrobiologie und Präventivmedizin", 1982

## Description

The invention is concerned with a pharmaceutical iodophor preparation and its manufacture. Apart from iodine with its longstanding disadvantages, iodophor preparations are known and commercially available which have considerable advantages over iodine, iodine tinctures etc. Attention is drawn, in particular, to polyvinylpyrrolidone iodine (PVP iodine), a preparation which is commercially available in a variety of forms and has recognizedly good microbicidal properties.

However, such iodophor preparations, including in particular PVP iodine, have also disadvantages which are important, above all, for their use, i.e. with regard to storage stability, reliable effectiveness over an extended period and reproducibility, i.e. their manufacture to a consistently good effective quality.

There has therefore been no shortage of experiments trying to eliminate these disadvantages. To this effect U.S. Patent No. 4,113,857 proposes a process for manufacturing such iodophor preparations, in which it is stressed, in particular, that the preparation does not contain any iodide, this being based on the recognition that the presence of iodide should be regarded as a contamination leading to a reduction of the free, elementary, caustic iodine. According to this patent it is therefore important to produce iodophor preparations which are free of iodide and are not contaminated by iodide ions.

Another experiment to improve the iodophor preparations is described in U.S. Patent No. 4,271,149, according to which preparations which are stable in storage are produced by the addition of iodate in an amount of 0.005% to 0.2%, preferably 0.05 to 0.1% and the control of the pH-value to within pH 5 to 7. In this specification great importance is attached to the iodide content for reasons of stability, as the concrete data show in the examples with the high iodide contents. This prior art aims at stabilizing the available, i.e. the titratable iodine level; no distinction is made between available and free iodine. There is no teaching the patent of free iodine and the iodophor preparations have proved not to provide sufficient free iodine levels over prolonged periods of time. Further, the preparation process of this prior art does not lead to stable free iodine levels immediately after preparation.

From the DE―OS 27 18 385 a biocide substance is known, for example PVP iodine, for which the presence of an oxidizing agent is essential in order to oxidize iodide to yield free iodine, which is then directly complex-bound, whereby the oxidizing agent prevents the reforming of iodide form iodine up to a stable balance. This target is not achieved.

From the DE―OS 24 38 594 a disinfectant is known, which for instance contains PVP iodine, but which contains hydrogen peroxide as an essential additive; this leads to an essentially reduced iodine content, but not to a stable preparation. In the FR―PS 745 693 a well known iodine bandage is described for which component ratios do not at all matter.

From the GB―A 2 024 012 a medical dressing is known which when applied to a wound gives off gaseous oxygen based on a concentration of a peroxide and decomposing agent suitable for it, for instance potassium iodide. Such dressing has nothing to do with the invention.

In the UP―PS 4,125,602 a process for manufacturing an iodophor is described which aims at an economical and quick cycle of operations. The process produces a granulate with uniform particle size. This publication does not contain any references as to how the disadvantages outlined in the beginning can be avoided.

It is accordingly a primary object of the present invention to produce iodophor preparations which avoid all of the disadvantages of known preparations, and which particularly provide predictable microbicidal activity over a prolonged period of time.

It is another object of the present invention to provide the method of producing such preparation.

It is yet a further object of the present invention to provide iodophor preparations which contain predetermined quantities of free iodine and which, due to the presence of such free iodine provide predictable microbicidal effectiveness over a prolonged period of time.

With the above and other objects in view, the present invention mainly comprises a pharmaceutical polyvinyl pyrrolidone (PVP)-iodine preparation comprising polyvinyl pyrrolidone (PVP)-iodine iodide ions and iodate ions, the preparation having a ratio of available iodine (both iodine bound to the polymer as a complex, and free iodine) to iodide between 2:1 to 10:1, preferably 2:1 to 6:1, and most preferably 2.1:1 to 3.6:1. The preparation has, in solution, a pH between 5 and 6 and a concentration between 2 and 20 ppm of free iodine, wherein the amount of the available iodine is at least 0.5 weight percent of the preparation, the concentration being stable for at least 12 months at 20°C. For the treatment of other than living beings, for example hospital corridors, etc., the pH value can be lowered to the acid range. In the preparation of the invention the presence of an oxidizing agent such as iodated ions in an amount of at least 0.22 weight % is advantageous for stabilizing the free iodine levels of the composition.

The levels of free iodine of the composition of the present invention are adjusted between 2―20 ppm, depending upon the use for which the preparation is to be put. The lower levels of free iodine are used for the treatment of living beings whereas higher levels free iodine are used for the treatment of inanimate objects.

The most important and most commonly used iodine complexing organic substance is polyvinyl-pyrrolidone also known as povidone.

The essential feature of the povidone-iodine preparation according to the present invention is the titratable iodine to iodide ratio, described subsequently also as internal ratio, and the free, non-complex-bound elementary iodine content resulting therefrom and from the large quantity of oxidizing agent such as iodate ions, which provides a predictable microbicidal effectiveness and duration of effectiveness. Thus, compared to prior iodophor preparations, it is possible to achieve a lower total iodine content, whilst the titratable iodine content is approximately comparable and the iodide content in the stated ratio is largely constant in relation to the titratable iodine content.

By having a fixed titratable iodine to iodide ratio during manufacture, a predictable iodine species content (with a certain bandwidth) of free, non-complex-bound elementary iodine is obtained in the solution responsible for the microbicidal effectiveness, due, for instance, to the chemical balance ratios in the solution between the iodine species and also between these and PVP (after a certain reaction period).

The advantages of these preparations according to the invention are astonishing: A predictable, quicker microbicidal effect as compared to previous preparations and a substantially increased sporocidal effect. Unusually short reaction times of approximately 15 seconds are achieved for the microbicidal effectiveness.

It is furthermore preferred that the total iodine content is 12 to 15 percent by weight relative to the polymer and that the titratable iodine content is 9 to 12 percent by weight relative to the polymer. It is additionally preferred that the oxidizing agent is contained in a quantity of 0.22 to 15, in particular 2 to 8 percent by weight. With this arrangement the preferred oxidizing agent is potassium iodate. It should be noted that the amount of oxidizing agent rises within the above mentioned limits the more the preparation is diluted. Preferably the preparation contains citric acid to buffer the pH-value, which may be mixed with a sodium phosphate for higher pH-values, preferably secondary sodium phosphate.

It was found that the preparation has a free, non-complex-bound elementary iodine content in solution of approximately 2 to 20 ppm I₂, preferably 2.5―3.6, for weaker action or 5―6 ppm I₂ for stronger action, measured by the potentiometric method of Gottardi (Fresenius Z Anal chem (1983), 582 to 585).

In the German Federal Republic chemical disinfection processes are tested and evaluated according to guidelines of the German Society for Hygiene and Microbiology (see Guidelines for the Testing and Evaluating of Chemical Disinfection Processes, issued January 1, 1981, printed in ISBN 3-437-10716-X). In the in-vitro tests (quantitative suspension experiments) mentioned in there it was found that a direct correlation existed between the free, non-complex-bound elementary iodine content and the reduction factors according to the guidelines within a specified time.

It is furthermore preferred that the preparation exists as a 0.1 to 10 percent by weight PVP iodine solution in water or an alcoholic solution or exists as liquid soap, ointment, gel, suppository or oral antiseptic with a 0.1 to 10 percent by weight PVP iodine content Accordingly the preferred application of the preparation according to the invention is as a microbicide such as solution, liquid soap, ointment, gel, suppository, antiseptic dressing, medicated dressing etc.

It has been found that the preferred titratable iodine to iodide ratio is 2:1 to 6:1. With this arrangement the free, non-complex-bound elementary iodine content is predominantly and in particular 2.5 to 3 or 5 to 6 ppm I₂ taking into account skin compatibility, microbicidal effectiveness and duration of reaction.

Manufacture of the preparations according to the invention may be effected in two ways, i.e. either by preparing a new fresh concentrate or by reprocessing old preparations, in particular old solution which no longer meet the practical requirements. Accordingly one may proceed by either a) dissolving the polymer in a polar carrier such as water and/or alcohol, then adding thoroughly dispersed iodine, possibly as a solution, while stirring at a moderately raised temperature, generally below 100°C, and permitting reaction to occur. A check is kept on the progress of the reaction by continuously analyzing the titratable iodine to iodide ratio. The iodine to iodide ratio is adjusted to have free iodine present for the desired killing effect As indicated above, the ratio of iodine to iodide for this purpose is 2:1―10:1, preferably 2:1―6:1, and most preferably 2.1:1 to 3.6:1. This ratio is adjusted by adding iodate ions while the solution is still in acid pH, in an amount of 0.01―0.45%, preferably 0.02―0.25%. The solution is analyzed to check the amount of free iodine. The amount of free iodine in the solution should be from about 1―20 ppm preferably 2―10 ppm. The amount of free iodine for weaker solutions is preferably between 2.5―3 ppm, and for stronger solutions preferably 5―6 ppm. For solutions to be used for inanimate objects, contents of 20 ppm and even higher can be used.

For use in connection with living beings, the pH of the solution is then increased to about 4―6.5, preferably 5―6, and most preferably 5.5―5.9, after which iodate ions are added in an amount of at least 0.2% to stabilize the free iodine level. The carrier can then be removed by spray-drying or freeze-drying or precipitation if temporary storage is required.

b) reprocessing an existing povidone-iodine preparation or the dissolved concentrate not corresponding to the set titratable iodine to iodide ratio by altering the titratable iodine to iodide ratio through the addition of an oxidizing agent such as iodate and constantly checking it analytically, and/or by determining the free non-complex-bound elementary iodine content, until the specified values have been obtained, whereupon the pH-value is adjusted using citric acid and soda lye, if necessary, and adding the oxidizing agent such as iodate, whereupon auxiliary substances, diluting agents, etc. are added, if necessary, in order to give the preparation the desired galenic form.

It is thus possible to produce, as required, a solid concentrate as intermediate product, or an aqueous solution, which may be used undiluted or without further galenic processing, or a galenic preparation such as solution etc., whereby it is up to the expert to add auxiliary substances and additives according to the requirement and objective.

The following examples are given to further illustrate the present invention.

### Control Example A (raw material)

Into a glass or stainless steel flask equipped with stirrer fill a solution made up from 100 g polyvinylpyrrolidone K 30 (Povidone K 30) and 1000 ml distilled water (acidified to a pH below 4). While stirring add 15.26 g potassium iodide. When this is dissolved add potassium iodate in small portions. 3.93 g potassium iodate is required for the complete removal of all iodide ions and the formation of Povidone iodine. Keep stirring until the test for iodide ions is negative.

After stirring for approximately one hour at room temperature the reaction will have stabilized. The solution is then left to stand overnight without stirring.

Now the carrier is extracted under reduced pressure. The Povidone iodine obtained is in the form of a brown-red, free-flowing powder; titratable iodine content approximately 10%, nitrogen content approximately 10.5%. The Povidone iodine obtained in this way fully meets the existing requirements of USP.

### Control Example B (raw material)

Into a glass or stainless steel flask equipped with stirrer fill a solution made up from 100 g Povidone K 30 and 1000 ml distilled water. While stirring add 15 g finely dispersed iodine in small portions. Stir for a further six hours at room temperature until the iodine is completely dissolved.

When the reaction has stabilized the solution is left to stand overnight without stirring.

Now the carrier is extracted under reduced pressure. The Povidone iodine obtained is in the form of a brown-red, free-flowing powder; titratable iodine content approximately 10%, nitrogen content approximately 10.5%. The Povidone iodine obtained in this way fully meets the existing requirements of USP.

### Control Example C (raw material)

Instead of Povidone K 30 Povidone K 90 may be used in the control Examples A and B. If in all other respects one proceeds as stated, Povidone iodine, an iodophor, is obtained, which differs, not in its anti-microbial effectiveness, but in its properties as regards solubility in aqueous carriers, from the products described there.

### Control Example D (raw material)

Instead of Povidone K 30 Povidone K 17 may be used in the control examples A and B. If in all other respects one proceeds as stated, Povidone iodine, an iodophor, is obtained, which differs, not in its anti-microbial effectiveness, but in its properties as regards solubility in aqueous carriers, from the products described there.

### Example 1 (preparation, iodine to iodide ratio)

Eight different PVP iodine preparations with differing iodine to iodide ratios were manufctured as follows: Batch 1―4: 10 g of PVP iodine containing 10% iodine were dissolved in 100 ml water at room temperature while stirring. Dependent upon the objective the desired iodine to iodide ratio was set up by the addition of potassium iodate as oxidizing agent or by potassium iodide. Then the pH value was adjusted by the addition of citric acid or soda lye. The individual batches had the following parameters:

| Batch | Titratable iodine | PVP content | Iodide content | I₂: iodide |
|---|---|---|---|---|
| 1 | 1.00% | 8.5% | 0.25% | 3.6:1 |
| 2 | 1.00% | 8.5% | 0.33% | 3.0:1 |
| 3 | 1.00% | 8.5% | 0.50% | 2.0:1 |
| 4 | 1.00% | 8.5% | 1.00% | 1.0:1 |

Batch 5―10: 5 g of PVP iodine containing 10% iodine were dissolved in 100 ml water at room temperature while stirring. Dependent upon the objective the desired iodine to iodide ratio was set up by the addition of potassium iodate as oxidizing agent or by potassium iodide. Then the pH-value was adjusted by the addition of citric acid or soda lye. The individual batches had the following parameters:

| Batch | Titratable iodine | PVP content | Iodide content | I₂: iodide |
|---|---|---|---|---|
| 5 | 0.50% | 4.25% | 0.125% | 3.6:1 |
| 6 | 0.50% | 4.25% | 0.16% | 3.0:1 |
| 7 | 0.50% | 4.25% | 0.25% | 2.0:1 |
| 8 | 0.50% | 4.25% | 0.50% | 1.0:1 |

A microbiological test was then carried out which revealed that preparations with an iodine to iodide ratio from approximately 3.0:1 are rapidly effective for practical use.

### Example 2 (preparation, iodine to iodide ratio)

100 ml of a one year old 10% solution of Povidone iodine in water containing 1.0 g titratable iodine and 0.5 g iodide (iodine to iodide ratio=2:1) are adjusted to a pH value of 2 and 0.056 g potassium iodate are added while stirring lightly. After approximately two hours the oxidation reaction has finished. A solution with 1.2 g titratable iodine and 0.34 g iodide has formed, in which regeneration has resulted in a 3.5:1 iodine to iodide ratio. The solution is adjusted to a pH value of 5.0 to 5.5 and stabilized by the addition of 0.2 g potassium iodate. It shows an excellent antimicrobial effectiveness.

### Example 3 (preparation, free iodine)

10 g PVP iodine containing 10% iodine were dissolved in 100 ml water at room temperature while stirring. Depending upon the objective a desired free iodine content of 5 ppm was obtained by the addition of potassium iodate as oxidation agent. Then the pH value was raised to 5.0―5.5 by the addition of citric acid and soda lye and the solution was stabilized by the addition of 0.2 g potassium iodate. The solution has an excellent antimicrobial effectiveness.

### Example 4 (preparation, stability over time)

The solution manufactured as per Example 3 (batch 9) was observed as regards stability of some relevant parameters (pH value, free iodine content, available iodine content) over a period of 12 months and compared with a conventionally prepared preparation (batch 10). As the table below shows, the solution manufactured by the new method has an excellent and substantially improved stability within the measured tolerance.

| Batch | storage period (months) | Available iodine % | Free iodine (ppm) | pH |
|---|---|---|---|---|
| 9 | 0 | 1.04 | 4.8 | 5.5 |
| | 3 | 1.04 | 4.9 | 5.5 |
| | 6 | 1.05 | 5.2 | 5.6 |
| | 9 | 1.05 | 4.9 | 5.5 |
| | 12 | 1.06 | 5.1 | 5.6 |
| 10 | 0 | 1.00 | 2.2 | 5.5 |
| | 3 | 0.98 | 2.0 | 5.2 |
| | 6 | 0.94 | 1.9 | 4.9 |
| | 9 | 0.91 | 1.5 | 4.6 |
| | 12 | 0.90 | 1.3 | 4.2 |

### Example 5 (preparation, internal ratio/reduction factors)

According to the "Guideline for the Testing and Evaluating of Chemical Disinfection Processes" by the German Society for Hygiene and Microbiology (DGHM) (Issue 1.1.1981) the bactericidal effect of various batches of 10% aqueous PVP-iodine-solutions with a titratable iodine to iodide ratio of 3.6:1 or 3:1 and containing 0.22% iodate was determined in a quantitative suspension experiment.
a) 3.6:1 ratio

| | Reduction factors RF at | | | |
|---|---|---|---|---|
| Batch | 30˝ | 1′ | 2′ | 5′ |
| a | 4.78 | 5.25 | 5.95 | ≥7.25 |
| b | ≥7.19 | ≥7.19 | ≥7.19 | ≥7.19 |
| c | 6.55 | 5.57 | 6.07 | ≥7.37 |
| d | 5.38 | 6.38 | ≥7.38 | ≥7.38 |

b) 3:1 ratio

| | Reduction | | factors RF at | |
|---|---|---|---|---|
| Batch | 30˝ | 1′ | 2′ | 5′ |
| e | 1.48 | 2.72 | 6.59 | ≥7.59 |
| f | 2.48 | 5.67 | 6.0 | ≥7.14 |
| g | 1.34 | 3.06 | 5.14 | ≥7.14 |
| h | 1.09 | 4.43 | ≥7.13 | ≥7.13 |

### Example 6

### (preparation, ppm, I₂-content/reduction factors)

According to Example 1 eight different batches with internal iodine to iodide ratios of 2.0:1 to 3.6:1 were manufactured. Of these batch was a 5% PVP iodine solution; all other batches were 10% PVP iodine solutions; with the exception of batch i all batches were stabilized with potassium iodate. In order to establish the correlation between microbicidal speed of effect and free elementary iodine content, the batches, differing in age, were microbiologically tested over the same period in a quantitative suspension experiment according to the DGHM method and their free elementary iodine content determined by the potentiometric method of Gottardi:

| | Reduction factors RF at | | | | |
|---|---|---|---|---|---|
| Batch | 30˝ | 1′ | 2′ | ppm I₂ | I₂:iodide |
| i | 0.94 | 1.15 | 2.18 | 1.91 | 2.0:1 |
| j | 4.97 | ≤7.50 | ≥7.50 | 4.97 | 2.4:1 |
| k | 5.31 | ≥7.17 | ≥7.17 | 4.90 | 3.0:1 |
| l | ≥7.50 | ≥7.50 | ≥7.50 | 5.80 | 3.0:1 |
| m | ≥7.50 | ≥7.50 | ≥7.50 | 6.39 | 3.0:1 |
| n | ≥7.21 | ≥7.21 | ≥7.21 | 6.46 | 3.0:1 |
| o | ≥7.50 | ≥7.50 | ≥7.50 | 12.70 | 3.6:1 |
| p | ≥7.41 | ≥7.41 | ≥7.41 | 16.01 | 3.6:1 |

These values show a distinct correlation between ppm I₂ and the RF. It is thus possible to control the desired sterilization times by means of ppm I₂ or internal ratio.

Based on these results stable batches of a 10% PVP iodine solution were manufactured, which easily reach the time value of 2 minutes, previously critical for conventional PVP iodine solutions, at the level of the minimum specified reduction factor in the quantitative suspension test against staphylococcus aureus by the DGHM method, even in the concentrated solution.

| | Reduction factors RF at | | | | | |
|---|---|---|---|---|---|---|
| Batch | 30˝ | 1′ | 2′ | ppm I₂ | I₂:iodide | pH |
| r | 2.26 | 4.40 | ≥8.30 | 4.35 | 2.4:1 | 5.9 |
| s | 3.16 | 5.17 | ≥7.91 | 4.50 | 2.4:1 | 5.9 |

## Claims

1. A method of producing a pharmaceutical polyvinylpyrrolidone (PVP) iodine preparation having a predetermined, constant concentration of free iodine, predictable microbicidal effectiveness and long duration of action, the method comprising the steps of
a) treating a mixture of PVP-iodine and iodide ions in acidic solution with iodate ions to form an acidic solution containing between 2 and 20 ppm of free iodine and having an available iodine to iodide ratio between 2 : 1 and 10 : 1;
b) increasing the pH of acidic solution to between 5 and 6, and
c) adding an amount of additional iodate ions to the thus formed solution, sufficient to maintain the amount of free iodine in the solution at a concentration between 2 and 20 ppm.

2. The method according to claim 1, wherein the acidic solution has an available iodine to iodide ratio between 2 : 1 and 6 : 1.

3. The method according to claim 2, wherein the ratio is between 2.1 : 1 and 3.6 : 1.

4. The method according to any one of claims 1 through 3, wherein the total amount of iodate ions added is at least 0.22 weight percent of the preparation.

5. A pharmaceutical polyvinylpyrrolidone (PVP)-iodine preparation producible by the method of anyone of claims 1 through 4, comprising polyvinylpyrrolidone iodine, iodide ions and iodate ions, the preparation having a ratio of available iodine to iodide between 2 : 1 and 10 : 1, and, in solution, a pH between 5 and 6, and a concentration between 2 and 20 ppm of free iodine, the concentration being stable for at least 12 months at 20° C, wherein the amount of available iodine is at least 0.5 weight percent of the preparation.

6. The preparation according to claim 5, wherein the available iodine to iodide ratio is between 2 : 1 and 6 : 1.

7. The preparation according to claim 6, wherein the ratio is between 2.1 : 1 and 3.6 : 1.

8. The preparation according to any one of claims 5 through 7, wherein the total amount of iodate ions added is at least 0.22 weight percent of the preparation.

9. The preparation according to any one of claims 5 through 8, the preparation having in solution a pH-value between 5.5 and 5.9.

10. The preparation according to any one of claims 5 through 9, wherein the available iodine content is between 9 and 12 weight percent of the polyvinylpyrrolidone weight.

11. The preparation according to claim 10, wherein the weight ratio of polyvinylpyrrolidone to available iodine is 8.5 : 1.

## Patentansprüche

1. Verfahren zur Herstellung einer pharmazeutischen Polyvinylpyrrolidon-(PVP)-Jod-Zusammensetzung mit einer vorgewählten konstanten Konzentration von freiem Jod, vorhersagbarer mikrobizider Wirksamkeit und langer Wirkungsdauer, wobei das Verfahren folgende Schritte umfaßt:
a) Behandlung einer Mischung aus PVP-Jod und Jodidionen in saurer Lösung mit Jodationen zur Bildung einer sauren Lösung, die zwischen 2 und 20 ppm freies Jod enthält und ein Verhältnis von verfügbarem Jod zu Jodid zwischen 2:1 und 10:1 hat;
b) Anhebung des pH-Wertes der sauren Lösung auf zwischen 5 und 6, und
c) Zugabe einer Menge zusätzlicher Jodationen zu der so gebildeten Lösung, die ausreicht, die Menge an freiem Jod in der Lösung bei einer Konzentration zwischen 2 und 20 ppm zu halten.

2. Verfahren nach Anspruch 1, bei dem die saure Lösung ein Verhältnis von verfügbarem Jod zu Jodid zwischen 2:1 und 6:1 hat.

3. Verfahren nach Anspruch 2, bei dem das Verhältnis zwischen 2,1:1 und 3,6:1 liegt.

4. Verfahren nach einem der Ansprüche 1 bis 3, bei dem die gesamte Menge der zugesetzten Jodationen wenigstens 0,22 Gew.-% der Zusammensetzung ausmacht.

5. Pharmazeutische Polyvinylpyrrolidon-(PVP)-Jod-Zusammensetzung, herstellbar nach dem Verfahren gemäß einem der Ansprüche 1 bis 4, welche Polyvinylpyrrolidon-Jod, Jodidionen und Jodationen umfaßt, wobei die Zusammensetzung ein Verhältnis von verfügbarem Jod zu Jodid zwischen 2:1 und 10:1 hat und in Lösung einen pH-Wert zwischen 5 und 6 hat und eine Konzentration zwischen 2 und 22 ppm an freiem Jod aufweist, welche Konzentration über wenigstens zwölf Monate bei 20°C stabil ist, bei der die Menge an verfügbarem Jod wenigstens 0,5 Gew.-% der Zusammensetzung ausmacht.

6. Zusammensetzung nach Anspruch 5, bei der das Verhältnis von verfügbarem Jod zu Jodid zwischen 2:1 und 6:1 liegt.

7. Zusammensetzung nach Anspruch 6, bei der das Verhältnis zwischen 2,1:1 und 3,6:1 liegt.

8. Zusammensetzung nach einem der Ansprüche 5 bis 7, bei der die gesamte Menge an zugesetzten Jodationen wenigstens 0,22 Gew.-% der Zusammensetzung ausmacht.

9. Zusammensetzung nach einem der Ansprüche 5 bis 8, wobei die Zusammensetzung in Lösung einen pH-Wert zwischen 5,5 und 5,9 hat.

10. Zusammensetzung nach einem der Ansprüche 5 bis 9, bei der der Gehalt an verfügbarem Jod zwischen 9 und 12 Gew.-%, bezogen auf das Gewicht des Polyvinyl-pyrrolidons ist.

11. Zusammensetzung nach Anspruch 10, bei der das Gewichtsverhältnis von Polyvinylpyrrolidon zu verfügbarem Jod 8,5:1 ist.

## Revendications

1. Procédé pour obtenir une préparation pharmaceutique polyvinylpyrrolidone (PVP)-iode possédant une concentration en iode libre constante prédéterminée, une activité microbicide prévisible et une action de longue durée, ce procédé comprenant les étapes consistant à:
a) traiter un mélange d'un constituant PVP-iode et d'ions iodures dans une solution acide avec des ions iodates pour former une solution acide contenant entre 2 et 20 ppm d'iode libre et possédant un rapport iode disponible à iodures entre 2:1 et 10:1;
b) accroître le pH de la solution acide entre 5 et 6, et
c) additioner une quantité d'ions iodates supplémentaires à la solution ainsi formée, suffisante pour maintenir la quantité d'iode libre dans la solution à une concentration entre 2 et 20 ppm.

2. Procédé selon la revendication 1, dans lequel la solution possède un rapport iode disponible à iodures entre 2:1 et 6:1.

3. Procédé selon la revendictation 2, dans lequel le rapport est compris entre 2,1:1 et 3,6:1.

4. Procédé selon l'une quelconque des revendications 1 à 3, dans lequel la quantité totale d'ions iodates additionnée est d'au moins 0,22 % en poids de la préparation.

5. Préparation polyvinylpyrrolidone (PVP)-iode pouvant être obtenue par le procédé selon l'une quelconque des revendications 1 à 4, comprenant un constituant polyvinyl-pyrrolidone-iode, des ions iodures et des ions iodates, la préparation possédant un rapport de l'iode disponible aux iodures entre 2:1 et 10:1 et, en solution, un pH entre 5 et 6, et une concentration en iode libre entre 2 et 20 ppm, la concentration étant stable pendant au moins 12 mois à 20°C, dans laquelle la quantité d'iode disponible est d'au moins 0,5 % en poids de la préparation.

6. Préparation selon la revendication 5, dans laquelle le rapport iode disponible à iodures est compris entre 2:1 et 6:1.

7. Préparation selon la revendication 6, dans laquelle le rapport est compris entre 2,1:1 et 3,6:1.

8. Préparation selon l'une quelconque des revendications 5 à 7, dans laquelle la quantité totale d' ions iodates additionée est d'au moins 0,22 % en poids de la préparation.

9. Préparation selon l'une quelconque des revendications 5 à 8, la préparation possédant en solution une valeur de pH entre 5,5 et 5,9.

10. Préparation selon l'une quelconque des revendications 5 à 9, dans laquelle la teneur en iode disponible est comprise entre 9 et 12 % en poids du poids de la polyvinylpyrrolidone.

11. Préparation selon la revendication 10, dans laquelle le rapport en poids de la polyvinylpyrrolidone à l'iode disponible est de 8,5:1.
